(19) Europäisches Patentamt / European Patent Office / Office européen des brevets

(11) **EP 4 091 638 A1**

(12) **EUROPEAN PATENT APPLICATION**
published in accordance with Art. 153(4) EPC

(43) Date of publication:
**23.11.2022 Bulletin 2022/47**

(21) Application number: **21740768.3**

(22) Date of filing: **11.01.2021**

(51) International Patent Classification (IPC):
**A61L 27/04** (2006.01)     **A61C 8/00** (2006.01)
**A61K 6/84** (2020.01)

(52) Cooperative Patent Classification (CPC):
**A61C 8/00; A61K 6/84; A61L 27/04**

(86) International application number:
**PCT/KR2021/000354**

(87) International publication number:
**WO 2021/145629 (22.07.2021 Gazette 2021/29)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.01.2020 KR 20200006226
04.01.2021 KR 20210000548**

(71) Applicant: **Kolon Industries, Inc.
Seoul 07793 (KR)**

(72) Inventors:
• **KIM, Choongnyun Paul
Seoul 07793 (KR)**
• **HAM, Gi Su
Seoul 07793 (KR)**

(74) Representative: **Ter Meer Steinmeister & Partner
Patentanwälte mbB
Nymphenburger Straße 4
80335 München (DE)**

(54) **AMORPHOUS METAL FOAM AND METHOD FOR PRODUCING SAME**

(57) One aspect of the present invention provides metal foam comprising pores, the amorphous metal foam comprising: a powder-connective bodies in which at least a portion of adjacent amorphous alloy powder is connected to form a networked structure; and a plurality of pores provided between the powder-connective bodies.

EP 4 091 638 A1

**Description**

[Technical Field]

An aspect of the present disclosure relates to an amorphous metal porous body and a method for producing the same, and more particularly, to a metal porous body containing an amorphous alloy and a method for producing the same.

[Background Art]

A porous metal refers to a metal having many pores formed therein, and is used in various fields such as aviation and electrochemical fields because it is ultralight and has excellent mechanical properties due to its unique structure with a high specific surface area.

The properties of the porous metal may vary depending on a porosity. In order to indicate the porosity, a porosity percentage indicating a volume ratio of pores or a relative density ($\rho\sim = \rho_2/\rho_1$) obtained by dividing a density ($\rho_2$) of a material having many pores by a density ($\rho_1$) of a dense material having no pore is used.

A relative density of a polymer material used for a cushion, a packing material, an insulation material, and the like is about 0.05 to 0.2. When a relative density of a metal is about 0.3 or less, pores have a cellular structure, and thus, the metal may be referred to as a metallic foam.

When the relative density is 0.3 or more, it transitions to a porous structure. Therefore, a metal having a porosity percentage of 70% or less may be technically defined as a porous metal. However, in general, a metal having a cellular structure with a high porosity percentage is also referred to as a porous metal.

The porous metal was first produced by Sosnik in 1948 by a method of adding mercury to molten aluminum to generate bubbles. Currently, various methods have been put into practical use in response to the use of the porous metal. As a representative production method, a melt gas injection method, a melt foaming method, an investment casting method, a metal deposition method, a unidirectional solidification method, a hollow metal sphere sintering method, a combustion synthesis method, a pressure sintering method, and the like have been reported.

There are types of pores of the porous metal, such as an open pore type in which pores are connected to each other, a closed pore type in which each pore is isolated, and a lotus-type pore type in which pores are stretched in one direction. The shape, pore size, and relative density of the pore, which are characteristic factors of the porous metal, may vary depending on the production method, and a suitable production method is selected and used according to the intended use.

Since the porous metal has a high porosity percentage, general properties thereof, such as physical, mechanical, and thermal properties, are significantly different from those of a non-porous material. Therefore, the porous metal exhibits functionalities, that cannot be expected from a general metal, such as excellent lightweightness, a high specific strength, sound absorbability and a vibration-proof ability by energy absorbability, an insulation ability by internal pores, a heat transfer ability by through pores, and reaction promotion by a large surface area.

In particular, a porous amorphous metal has high mechanical strength to a low density due to excellent physical and chemical properties of an amorphous alloy powder particle itself, may be used as a functional material such as an energy absorbent or a fuel cell because it has a unique porous particle structure, and may be used as a gas and liquid filtration filter when being produced an open pore foam. A casting method in which casting is performed with a porous foam or a powder particle metallurgy method in which powder particles or metal fibers are sintered is mainly used.

An ultra-light material, a shock absorbing material, a vibration absorbing material, a soundproof material, an insulation material, a filter material, a heat exchange material, a biomedical material, and the like have been developed by utilizing the properties of the porous amorphous metal. Applied products thereof have been released in a wide range of fields such as the construction industry, the automobile industry, the machinery industry, the electronics industry, the environmental industry, and the energy industry, and studies on the porous amorphous metal have been actively conducted until recently. However, there are limits to controlling the size and the distribution of the pores according to the production method and to producing a product having uniform properties, and available metals are also mostly limited to aluminum or zirconium and alloys thereof. Therefore, there is a need for further development of fields in which the porous amorphous metal may be used by being applied to metals or alloys of various compositions.

[Disclosure]

[Technical Problem]

An aspect of the present disclosure is devised to solve the limitations of the metal porous body according to the related art and the problems occurring in production of a metal porous body using an amorphous alloy, and is to provide a metal porous body having an improved quality by reducing variations in general physical properties of a product due to a constant uniformity and distribution of pores.

Another aspect of the present disclosure is to provide a metal porous body formed of an amorphous phase by using an amorphous alloy powder particle and inducing diffusion and melting of a portion of a surface of the amorphous alloy powder particle.

Another aspect of the present disclosure is to provide a metal porous body that is excellent in physical and chemical properties such as a hardness and corrosion resistance because it is formed of an alloy containing an amorphous phase.

[Technical Solution]

According to an aspect of the present disclosure, an amorphous metal porous body that is a metal porous body including pores includes:

powder particle connection bodies in which at least portions of amorphous alloy powder particles adjacent to each other are connected in a network structure; and
a plurality of pores provided between the powder particle connection bodies.

Here, the powder particle connection body may include a bonding portion (neck) at which portions of surfaces of the adjacent amorphous alloy powder particles are fused and bonded to each other,

the amorphous metal porous body may have a corrosion loss rate of 10.0% or less under a condition of 50% HF, and
the amorphous alloy powder particle may be connected to adj acent alloy powder particles at at least two bonding portions to form the network structure.

In addition, a hardness of the amorphous metal porous body may be 700 to 1,300 Hv, a density of the amorphous metal porous body may be 1.5 to 7.0 g/cm$^2$, and
a uniformity of the pores is preferably 1 to 30.

In addition, the amorphous alloy powder particle may include an iron-based alloy having at least one element selected from the group consisting of Cr, Mo, Mn, Co, Cu, Al, Ti, V, Si, Ni, P, Zn, Zr, Nb, Ag, Ta, Mg, Sn, W, Y, B, and C, and Fe, and
the amorphous alloy powder particles may include first and second amorphous alloy powder particles having different average particle diameters.

According to another aspect of the present disclosure, a method for producing an amorphous metal porous body includes: a powder particle preparation step of preparing amorphous alloy powder particles containing amorphous phases; and
a sintering step of supplying an input energy to the amorphous alloy powder particles to form powder particle connection bodies in which amorphous alloy powder particles adjacent to each other are connected in a network structure.

Here, the input energy may be an electric energy,

the powder particle connection body may include the amorphous alloy powder particles that are adj acent to each other and have portions of surfaces connected to each other at an outer face, and
a distance (d) between the centers of the amorphous alloy powder particles may satisfy the following Expression 1:

$$(\text{Expression 1})$$
$$0.5 \times (D_A + D_B)/2 \leq d \leq 1.1 \times (D_A + D_B)/2$$

(wherein each of $D_A$ and $D_B$ is a particle diameter of each of the adjacent amorphous alloy powder particles).

In addition, the amorphous alloy powder particles may include first and second amorphous alloy powder particles having different average particle diameters, and
the input energy may be 0.1 to 0.5 kJ.

According to another aspect of the present disclosure, a method for producing an amorphous metal porous body includes: a powder particle preparation step of preparing amorphous alloy powder particles containing amorphous phases; and
a sintering step of applying a temperature and a pressure to the amorphous alloy powder particles to form powder particle connection bodies in which amorphous alloy powder particles adjacent to each other are connected in a network structure.

The powder particle connection body may include the amorphous alloy powder particles that are adj acent to each other and have portions of surfaces connected to each other at an outer face, and

a distance (d) between the centers of the amorphous alloy powder particles may satisfy the following Expression 1:

$$(\text{Expression 1})$$

$$0.5 \times (D_A + D_B)/2 \leq d \leq 1.1 \times (D_A + D_B)/2$$

(wherein each of $D_A$ and $D_B$ is a particle diameter of each of the adjacent amorphous alloy powder particles).

In addition,

the amorphous alloy powder particles may include first and second amorphous alloy powder particles having different average particle diameters,
in the sintering, the temperature may be 400°C or higher and 1,200°C or lower, and the pressure may be 10 MPa or more and 100 MPa or less,
the powder particle preparation step
preferably further includes a step of mixing space holders having a composition different from that of the amorphous alloy powder particle with the amorphous alloy powder particles, and
the space holders
preferably include one or two or more materials selected from the group consisting of polyethylene, polypropylene, polystyrene (PS), ethylene vinyl acetate, polyoxymethylene, carbohydrate, ammonium carbonate, carbamide, a metal, and an inorganic metal salt.

[Advantageous Effects]

As set forth above, the metal porous body according to an aspect of the present disclosure has the structure in which the amorphous alloy powder particles are connected to each other at the bonding portion (necks) on the surfaces, has excellent physical properties of the amorphous alloy, such as corrosion resistance and a hardness, and has an excellent specific strength compared to a crystalline metal porous body.

Further, a powder particle foam may be produced by melting the portions of the surfaces without completely melting the amorphous alloy powder particles, such that it is possible to prevent formation of a crystalline phase due to an insufficient cooling rate when cooling the amorphous alloy. Therefore, a metal porous body having excellent physical and chemical properties of the amorphous alloy may be produced.

Further, in a case where the amorphous alloy powder particles are stacked in contact with each other, voids formed between the powder particles may be formed in a uniform size and distribution in the entire volume. Thus, a plurality of pores may be formed by the uniformly distributed voids when the powder particle connection bodies are formed, and the physical properties are uniform due to excellent dispersion because the uniformity of the plurality of pores provided in the entire amorphous metal porous body is high.

Further, since the amorphous metal porous body may be produced into an open pore type foam by controlling the porosity percentage, the amorphous metal porous body may be applied to a gas filtration filter, and may be applied to an acidic gas filtration filter based on the excellent corrosion resistance of amorphous.

Further, the mechanical strength is set to a level similar to that of a human bone by controlling the pore size and the porosity percentage of the amorphous metal porous body, such that the amorphous metal porous body may be used as a biocompatible material that may be used as an implant or graft material for dental or orthopedic use.

[Description of Drawings]

FIG. 1 is a schematic view illustrating a structure of an amorphous metal porous body according to an aspect of the present disclosure.

FIG. 2 is a view illustrating a hexagonal closed packing structure, a face centered cubic structure, and a body-centered cubic structure, which are structures in which particles may be arranged in a space.

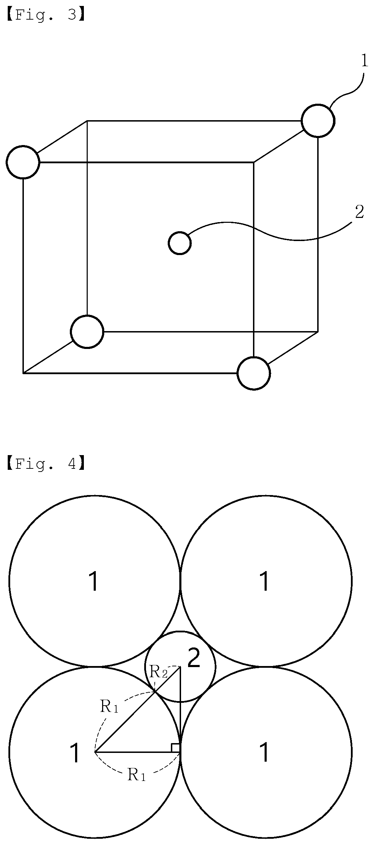

FIG. 3 is a three-dimensional view illustrating an arrangement between particles in a tetrahedral site in a case where particles having difference sizes are mixed and filled.

FIG. 4 is a schematic view illustrating a relationship between an arrangement and a radius between particles on a plane in a case where particles having different sizes are mixed and filled.

[Best Mode for Invention]

Prior to describing the present disclosure in detail below, it should be understood that the terms used in the present specification are merely intended to describe specific exemplary embodiments and are not to be construed as limiting the scope of the present disclosure, which is defined by the appended claims. Unless otherwise defined, all the technical terms and scientific terms used in the present specification have the same meanings as commonly understood by those skilled in the art to which the present disclosure pertains.

Throughout the present specification and the claims, unless otherwise defined, the terms "comprise", "comprises", and "comprising" will be understood to imply the inclusion of a stated object, a step or group of objects, and a step, but not the exclusion of any other objects, steps or groups of objects, or steps.

Meanwhile, unless clearly indicated to the contrary, various exemplary embodiments in the present disclosure may be combined with any other exemplary embodiments. In particular, any feature which is mentioned preferably or favorably may be combined with any other features which may be mentioned preferably or favorably. Hereinafter, exemplary embodiments in the present disclosure and effects thereof will be described in detail with reference to the accompanying drawings.

According to an aspect of the present disclosure, an amorphous metal porous body 100 is formed of an amorphous alloy, and the amorphous metal porous body 100 includes a plurality of pores 10 provided on a surface and inside thereof.

The amorphous alloy in the present specification is an alloy to which alloying elements that improve an amorphous forming ability are added, and has excellent chemical and mechanical properties because it has a short range ordered structure or a long range ordered structure depending on a cooling rate. Therefore, the amorphous alloy refers to a material having physical properties that are more excellent than those of a general crystalline alloy. In the present specification, the amorphous alloy is understood in a broad sense to mean an alloy containing an amorphous phase in at least one region according to a production method and a post-treatment method.

The amorphous metal porous body according to the aspect is formed of an amorphous powder particle having a significantly high amorphous forming ability or a semi-amorphous powder particle having a relatively lower amorphous forming ability than that of the amorphous powder particle, and is a metal porous body including the plurality of pores 10 provided between the powder particles.

The metal or alloy containing an amorphous phase in the metal porous body is not limited, but the metal porous body is preferably formed of an iron (Fe)-based amorphous alloy. According to an exemplary embodiment in the present disclosure, it is possible to use an alloy having a composition in which other elements contained in the amorphous alloy are substituted or included or content ratios of some elements are changed.

According to an exemplary embodiment in the present disclosure, an alloy composition of an amorphous alloy powder particle 1 may be an iron-based alloy containing at least one element selected from the group consisting of Cr, Mo, Co, Cu, Al, Ti, V, Si, Ni, P, Zn, Zr, Nb, Ag, Ta, Mg, Sn, W, Y, B, and C, and a balance of Fe, and in particular, an alloy having a composition having a high amorphous forming ability may be used.

Specifically, an alloy having a composition containing 30 to 50 wt% of at least one of Cr and Mo and 0.5 to 10 wt% of at least one selected from the group consisting of Si, B, and C, and further containing less than 40 wt% of at least one selected from the group consisting of Co, Cu, Al, Ti, V, Si, Ni, P, Zn, Zr, Nb, Ag, Ta, Mg, Sn, and W may be used.

As an example of the alloy, an alloy containing 20 to 35 wt% of Mo, 10 to 20 wt% of Cr, less than 1 to 7 wt% of at least one of Si, B, and C, and a balance of Fe may be used.

As another example, an alloy having a composition containing 20 to 40 wt% of Cr, 3 to 10 wt% of Mo, 1 to 7 wt% of at least one of Si, Bi, and C, and 0.5 to 35 wt% of at least one selected from the group consisting of Co, Cu, Al, Ti, V, Si, Ni, P, Zn, Zr, Nb, Ag, Ta, Mg, Sn, and W may be used.

As another example, a Co-Cr-based amorphous alloy, Ti-based amorphous alloy (Ti-Si-based, Ti-Zr-based, or Ti-Nb-based alloy, or the like), or Mg-based amorphous alloy (Mg-Zn-based or Mg-Cu) having excellent biocompatibility may be used. As further another example, a Ni-based amorphous alloy (Ni-W-based, Ni-Nb-based, Ni-Cr-based, Ni-Cu-based, or the like) having excellent high-temperature mechanical properties and corrosion resistance may also be used.

In a case where an Fe-based amorphous alloy does not satisfy the corresponding alloy composition, a fraction of an amorphous phase contained in the alloy is lowered, and thus, the effects such as physical properties of the amorphous alloy are not high, which causes degradation of physical and chemical properties (hardness, strength, corrosion resistance, and the like) of the metal porous body.

In a case where the metal porous body is produced using an amorphous alloy, it is possible to form the pores 10 by completely liquefying the alloy and then cooling the alloy again, and a significantly high cooling rate for cooling the metal porous body is required to have an amorphous phase. There is a limitation in that a size, a thickness, or the like of the metal porous body should be below a certain level to produce a metal porous body while satisfying such a cooling rate condition. However, in a case where an amorphous metal porous body is produced using an amorphous alloy under non-melting conditions, it is possible to obtain the amorphous metal porous body 100 in which the uniform pores 10 are provided while a high ratio of the amorphous phase is maintained by forming a powder particle connection body 4 without completely melting the amorphous alloy powder particle 1 containing the amorphous phase.

The amorphous metal porous body 100 includes the powder particle connection bodies 4 in which the amorphous alloy powder particles 1 having the compositions described above are three-dimensionally connected to each other to form a network structure, and the plurality of pores 10 provided between the powder particle connection bodies 4.

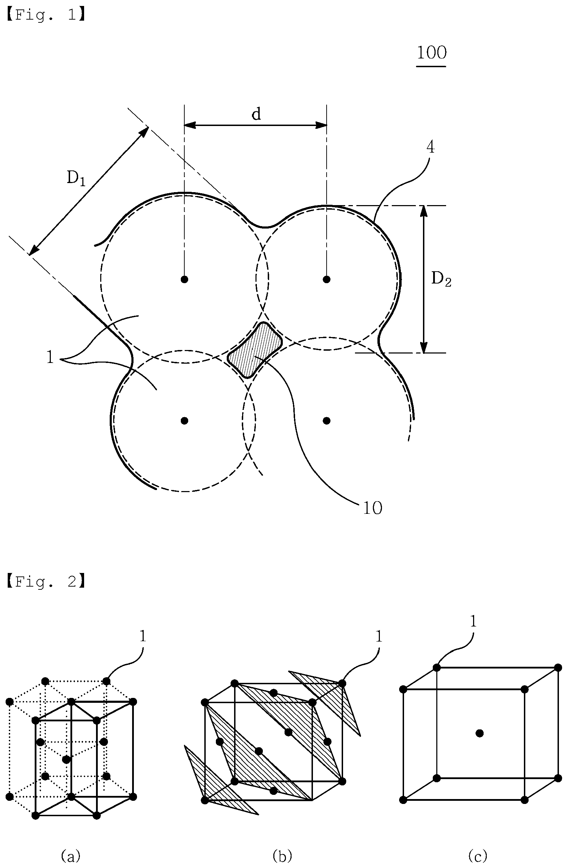

FIG. 1 is a cross-sectional view schematically illustrating a part of a structure of the amorphous metal porous body 100 according to an exemplary embodiment in the present disclosure, and illustrates the pore 10 provided between the powder particle connection body 4 and the powder particle connection body 4.

The powder particle connection body 4 (sintering neck) refers to a structure in which the amorphous alloy powder particles 1 are connected to at least two different other amorphous alloy powder particles 1 adjacent to each other at an outer face through bonding portions. The powder particle connection body 4 has a structure in which the amorphous alloy powder particle 1 is at least partially melted or softened on a surface to form a bonding portion with the adjacent amorphous alloy powder particle 1, and may include at least a part of the form of the amorphous alloy powder particle 1 because only a portion of the amorphous alloy powder particle 1 is melted.

One amorphous alloy powder particle 1 may form a bonding portion with at least one amorphous alloy powder particle 1 adjacent at the periphery thereof, and preferably, one amorphous alloy powder particle 1 may form bonding portions with at least two amorphous alloy powder particles 1 to form a three-dimensional network structure.

The amorphous metal porous body 100 may be produced without completely melting a powder particle by using the amorphous alloy powder particle 1 or a composite powder particle mixed material including the amorphous alloy powder particle 1. In this case, the adjacent amorphous alloy powder particle 1 may be a powder particle having a single particle size distribution, or may be a mixed powder particle having different average particle size distributions.

The amorphous alloy powder particles 1 having a particle diameter of $D_A$ and the other amorphous alloy powder particles 1 having a particle diameter of $D_B$ may form contact points or contact surfaces with each other, may form bonding portions with each other in a contact state, and may be connected to form the powder particle connection bodies 4. Here, $D_A$ and $D_B$ may be the same particle diameters, particles diameters of two arbitrary powder particles obtained from powder particles having a single particle size distribution, or particle diameters of powder particles included in a mixed powder particle of powder particles having different average particle diameters.

In this case, when defining a distance d between the centers of the powder particles with respect to a distance between two adjacent amorphous alloy powder particles 1, a powder particle center distance before sintering is represented by $d = (D_A + D_B)/2$.

The amorphous alloy powder particles 1 after sintering form the powder particle connection body 4 and form the bonding portion at the contact surface. When a distance between the centers of the amorphous alloy powder particles 1 is d, the distance d may satisfy 0.5 to 1.1 times and preferably 0.5 to 0.8 times the distance between the centers of the amorphous alloy powder particles 1 before forming the powder particle connection body 4, and may be represented by the following Expression 1:

$$\text{(Expression 1)}$$

$$0.5 \times (D_A + D_B)/2 \leq d \leq 1.1 \times (D_A + D_B)/2$$

(wherein each of $D_A$ and $D_B$ is a particle diameter of each of the adjacent amorphous alloy powder particles).

In a case where a range of d is smaller than the corresponding range, when the powder particle connection body 4 is formed, a shape deformation of the amorphous alloy powder particle 1 becomes too large, which may cause an increase in thickness of the amorphous bonding portion, and the particle diameter of the pore 10 is decreased, which may cause an increase in ratio of closed pores. In a case where the range of d is larger than the corresponding range, the thickness of the amorphous bonding portion becomes too thin, such that the adjacent pores 10 are connected to each other or the uniformity of the pores 10 is deteriorated, resulting in degradation of mechanical strength.

The amorphous alloy powder particle 1 may be a powder particle having a spherical shape or a substantially spherical shape, and in this case, when the spherical powder particles are stacked, inter-powder particle voids inevitably generated are uniformly distributed in the entire volume, and a size of the void is affected by the particle diameter and the particle size distribution of the amorphous alloy powder particles 1, but the voids may have a high uniformity.

In the amorphous metal porous body 100 according to an exemplary embodiment in the present disclosure, the voids formed in the stacking of the powder particles may be formed into the pores 10 provided between the powder particle connection bodies 4, such that the plurality of pores 10 provided in the amorphous metal porous body 100 are uniform and have a uniform size. Therefore, a pore uniformity is high.

FIG. 2 is a view illustrating various three-dimensional arrangements that the powder particles may have in a space.

Specifically, (a), (b), and (c) of FIG. 2 illustrate ideal packing methods or filling structures assuming spherical particles having the same diameter. Assuming that the amorphous alloy powder particles 1 are all spherical particles having the same diameter, the position of each point illustrated indicates a position of the center of the amorphous alloy powder particle 1 when the amorphous alloy powder particles 1 are filled. Although the surface and the appearance of the amorphous alloy powder particle 1 are not illustrated, the amorphous alloy powder particles 1 have a structure in which they are in contact with each other on the surfaces.

(a) and (b) of FIG. 2 are called a hexagonal closed packing (HCP) structure and a face centered cubic (FCC) structure, respectively, and have a filling rate of about 74%, and (c) of FIG. 2 is a body-centered cubic (BCC) structure and is a structure with a filling rate of about 68%.

Specifically, assuming that the amorphous alloy powder particles 1 form a hexagonal closed packing (hcp) structure or a face centered cubic structure, the filling rate in the space may be maximized, a coordination number, which is the number of adjacent amorphous alloy powder particles 1 in contact with one amorphous alloy powder particle 1, may be 12.

That is, in the case where the amorphous alloy powder particles 1 after being filled into the corresponding structure form the bonding portion and the powder particle connection body 4 with each other, one amorphous alloy powder particle 1 positioned inside the metal porous body may form bonding portions with about 12 powder particles on average.

Sizes, a density, and the like of the plurality of pores 10 included in the amorphous metal porous body 100 may be controlled, and physical properties such as a hardness and a modulus of elasticity may be changed. Therefore, use and application of the metal porous body may vary.

Meanwhile, the pores 10 included in the amorphous metal porous body 100 have a great influence on the properties of the amorphous metal porous body 100. The plurality of pores 10 included in the amorphous metal porous body 100 may be divided into open pores formed in a structure connected to the outside and closed pores formed in a structure not connected to the outside according to the structure thereof. The structure of the pores included in the amorphous metal porous body 100 according to an aspect of the present disclosure is not limited, the structure of the pores may vary depending on the sizes and the number of all the pores, and an amorphous metal porous body 100 including both open pores and closed pores may be utilized.

The pores 10 may have various shapes. For example, the shape of the pore 10 may be a substantially spherical shape to a substantially rectangular parallelepiped shape. A pore diameter of the pore 10 greatly affects the overall physical properties of the amorphous metal porous body 100, and when the pore diameter of the pore 10 is too large, the mechanical strength and the modulus of elasticity of the metal porous body may be degraded.

In order to indicate the size of the pore 10, the pore diameter of the pore is defined and used below. The pore diameter of the pore refers to a size of the pore observed on a cross section of the metal porous body. In a case of a spherical pore, when the diameter of the pore in the cross section and the shape of the pore in the cross section are out of a spherical shape, an average value of major and minor diameters is defined as a pore diameter of the pore. That is, in a case where the term "pore diameter of the pore" is used below, this is used to be meant to include the diameter of the spherical pore observed in the cross section of the amorphous metal porous body 100 and the average value of the major and minor diameters of the non-spherical pore.

As for the pore diameter of the pore 10, the average pore diameter and the porosity of the pores 10 of the amorphous metal porous body 100 according to an aspect of the present disclosure may vary depending on an application component, and may be variously controlled according to the technical field in which the amorphous metal porous body 100 is used or the physical properties and the performance required for the corresponding component.

As for the average pore diameter of the pores 10, when the amorphous metal porous body 100 is applied as a filter material as an open pore foam, the pore diameter of the pore may be 30 to 300 μm, preferably 30 to 200 μm, and more preferably 30 to 150 μm, and in a component requiring mechanical strength, the pore diameter of the pore may be 10 to 300 μm, preferably 10 to 200 μm, and more preferably 10 to 100 μm.

In a case where the amorphous metal porous body 100 is used as a filter material, when the average pore diameter of the pores is smaller than the corresponding range, the pores are clogged or a flow rate of a fluid is lowered, which may cause a reduction in efficiency of treatment with the filter, and when the average pore diameter of the pores is larger than the corresponding range, filtering efficiency may be reduced.

In a case where the amorphous metal porous body 100 is used as a component or graft material requiring mechanical strength, when the average pore diameter of the pores is less than 10 μm, a density may be increased and a specific strength may be degraded, and when the average pore diameter of the pores is larger than the corresponding range, properties such as a hardness, a yield strength, and a breaking strength may be degraded.

When the uniformity of the pores is defined as a value obtained by dividing a pore diameter of the pore having the largest pore diameter among the plurality of pores 10 (maximum pore diameter) by a pore diameter of the pore having the smallest pore diameter among the plurality of pores 10 (minimum pore diameter), the uniformity of the pores of the amorphous metal porous body 100 according to an aspect of the present disclosure may be 1 to 30.

Specifically, in the case of the amorphous metal porous body 100 including open pores, it is effective that the uniformity of the pores is 5 to 30, preferably 10 to 20, and more preferably 10 to 15, and when mechanical strength is required, the uniformity of the pores may be 1 to 30, preferably 1 to 20, and more preferably 1 to 10.

In a case where the uniformities of the pore diameters of the pores and the sizes of the pores are out of this range, when the amorphous metal porous body 100 is applied as a filter material, a flow rate of gas and liquid is significantly reduced due to clogging of the pores, such that the filtering effect may not be obtained. In applications requiring mechanical strength, breakage and loss of the metal porous body may occur due to degradations of the hardness, the yield strength, and the breaking strength, which may cause a decrease in productivity.

Meanwhile, the uniformity of the pores is a factor affecting a volume fraction and a distribution of the included pores or the porosity percentage of the amorphous metal porous body 100, in addition to the size of the pore, and the volume fraction occupied by the pores may vary depending on the size of the pore described above, and may be controlled differently depending on the purpose, use, and the like of the metal porous body.

For example, the volume fraction or porosity percentage occupied by all the pores 10 in the entire foam may be 5 to 30%, preferably 5 to 20%, and more preferably 5 to 10%, when the metal porous body is used as a component or graft material requiring mechanical strength, and may be 50 to 90%, preferably 50 to 80%, and more preferably 50 to 70%, when the metal porous body is used as a filter material (open pore foam).

When the volume fraction or porosity percentage occupied by all the pores 10 included in the amorphous metal porous body 100 is out of the corresponding range, the efficiency or utilization of the filter may be reduced due to formation of closed pores and degradation of the mechanical strength, the performance of the component may be deteriorated, or cost-effectiveness may be reduced.

Meanwhile, a bonding portion at which the amorphous alloy powder particles 1 are connected to each other is provided between the adjacent pores 10 that are not connected to each other . The bonding portion may serve to maintain the mechanical strength of the amorphous metal porous body 100 between the adjacent pores 10, may serve to support the foam, or may serve to couple the foam to a component, and may serve as a filter for removing solid foreign substances from gas and liquid.

A thickness or a width of the bonding portion has the same meaning as an average distance between the pores 10 dispersed in a matrix, and the average distance between the pores 10 dispersed in the amorphous metal porous body 100 may be 10 to 100 $\mu$m and preferably 30 to 90 $\mu$m.

When the average distance between the pores 10 is out of this range, closed pores are formed or the mechanical strength is degraded, which causes breakage of the component. Thus, cost-effectiveness may be reduced.

The amorphous metal porous body 100 according to an aspect of the present disclosure may have unique physical properties different from those of a general metal molded body or the like, and may have excellent properties such as a hardness to a density and a compressive strength, and chemical properties of the amorphous alloy powder particle are also excellent.

Specifically, the amorphous metal porous body 100 according to an exemplary embodiment in the present disclosure may have various densities according to the porosity percentage, and the density of the amorphous metal porous body 100 is 4.0 to 7.0 and preferably 5.0 to 7.0 (g/cm$^3$) when being used as a structural material. When the density is out of the corresponding density range, a degradation of strength may occur due to an increase in porosity percentage, the cost-effectiveness may be reduced as a weight is increased, and durability may be deteriorated due to a degradation of the mechanical strength.

In addition, a foam including open pores is used as a filter material, a density is 1.5 to 3.5 and preferably 2.5 to 3.5 (g/cm$^3$), and when the density is out of the corresponding density range, a flow rate of fluid and gas is reduced due to a phenomenon in which the pores are closed, or the function of the filter is lost.

An iron-based amorphous metal porous body 100 may utilize excellent physical and chemical properties of an iron-based amorphous alloy powder particle 1, for example, a hardness, a yield strength, and corrosion resistance, and has excellent corrosion properties, particularly in an acidic atmosphere.

The corrosion properties in an acidic atmosphere may be confirmed by a loss rate due to corrosion, and the loss rate is calculated as a ratio of a weight after a corrosion test to a weight before the corrosion test.

The loss rate may vary depending on the type of acid used, and for example, acids such as hydrofluoric acid, hydrochloric acid, and sulfuric acid may be used.

For example, a corrosion loss rate in a hydrofluoric acid (HF) atmosphere (weight after corrosion test/weight before corrosion test * 100) of the amorphous metal porous body 100 according to an aspect of the present disclosure may be 1 to 15%, preferably 1 to 10%, more preferably 1 to 5%, and still more preferably 1 to 3%.

In addition, a corrosion loss rate in a hydrochloric acid (HCl) atmosphere may be 0.0 to 10.0%, preferably 0.0 to 5.0%, and more preferably 0.0 to 2.0%.

In addition, a corrosion loss rate in a sulfuric acid ($H_2SO_4$) atmosphere may be 0.01 to 1.0%, preferably 0.01 to 0.4%, and more preferably 0.01 to 0.2%.

When the corrosion loss rate of the amorphous metal porous body 100 is out of the corresponding range, the purity of gas and liquid to be filtered may be reduced, it is difficult to apply to an ultra-high-purity filtration filter, and it is difficult to support the structure of the foam due to corrosion of an alloy strut or neck, and thus, the cost-effectiveness may be reduced due to breakage of the component.

Meanwhile, the hardness of the amorphous metal porous body 100 is ideal as similar to the hardness of the amorphous alloy powder particle 1 added, and may be 700 to 1,300 Hv, preferably 800 to 1,300 Hv, and more preferably 900 to 1,300 Hv.

When the hardness of the amorphous metal porous body 100 does not satisfy the corresponding range, the component may be broken when being fastened or cracks may be formed due to wear during use, which may cause a decrease in productivity due to a replacement of the component.

The metal porous body according to an aspect of the present disclosure is the amorphous metal porous body 100 satisfying all the properties described above. Specifically, it is preferable that the metal porous body includes pores having a pore diameter or porosity percentage in a specific range in order to have the density in the range described above. In this case, the metal porous body having the density in the corresponding range may have a corrosion loss rate in the range described above.

That is, the physical properties of such a metal porous body are factors that are organically connected to each other, and when one or more of the factors described above are out of the ranges described above, other properties of the metal porous body are degraded or the original function is lost, and thus, the object of the present disclosure may not be achieved.

The amorphous metal porous body 100 according to an aspect of the present disclosure may be used in various fields because it has the physical properties in the ranges described above. The application of the amorphous metal porous body 100 is not limited, and the amorphous metal porous body 100 may be applied to automobile interior materials, shock-absorbing materials, sound-absorbing materials, gas and liquid filter materials, catalysts or implants, graft materials such as artificial bones and joints utilizing lightweight and high-strength properties.

In particular, in a case where the metal porous body according to an aspect of the present disclosure is used as a medical material, the metal porous body may be used instead of a cobalt-chromium alloy or tantalum-based or zirconium-based alloy having been used in the related art, may promote growth of tissues because it has the pore diameter of the pore described above and a modulus of elasticity similar to that of a bone when being implanted in vivo, and may improve biocompatibility and stability.

[Mode for Invention]

Another aspect of the present disclosure relates to a method for producing an amorphous metal porous body 100. Hereinafter, the method for producing an amorphous metal porous body 100 having the structure described above will be described.

An example of the present aspect is a method for producing an amorphous metal porous body 100 by sintering an amorphous alloy powder particle by an electro-discharge-sintering method. Hereinafter, contents that are the same as the contents described above may be included in the specification, and some repeated contents have been omitted.

The method for producing an amorphous metal porous body 100 by electro-discharge-sintering includes an amorphous alloy powder particle preparation step S11 and a sintering step S12.

The amorphous alloy powder particle preparation step is a step of preparing amorphous alloy powder particles 1 used in the amorphous metal porous body 100. The metal porous body is produced using the amorphous alloy powder particles 1. It is preferable that an amorphous alloy having the same composition as the amorphous metal porous body 100 described above, specifically, an Fe-based amorphous alloy is used, and in this case, it is preferable to use an Fe-based amorphous alloy prepared in the form of a powder particle.

It is preferable to use a powder particle having a substantially spherical shape, and in this case, the prepared amorphous alloy powder particles 1 have inter-powder particle voids provided between the spherical powder particles by themselves when being filled in a predetermined space or volume.

The amorphous alloy powder particles 1 may be a group of powder particles having a uniform particle diameter or a unimodal particle diameter distribution, and may include first and second amorphous alloy powder particles having different particle diameters.

In a case where powder particles having a uniform particle diameter are used, an average particle diameter of the amorphous alloy powder particles 1 used may be 30 to 160 $\mu$m, preferably 30 to 130 $\mu$m, and more preferably 30 to 100 $\mu$m.

In a case where the first and second amorphous alloy powder particles that are the amorphous alloy powder particles having different particle diameters are used, an average particle diameter D1 of the first amorphous alloy powder particles may be 30 to 160 $\mu$m, preferably 30 to 130 $\mu$m, and more preferably 30 to 100 $\mu$m, and the second amorphous alloy powder particles have an average particle diameter D2 smaller than that of the first amorphous alloy powder particles. Specifically, D2 may be 30 to 60 $\mu$m and preferably 30 to 50 $\mu$m.

When the size of the amorphous alloy powder particle is out of the corresponding range, the inter-powder particle voids provided in the entire volume are closed to form closed pores, or it is difficult to variously disperse the voids, and thus, non-uniform shape, size, and distribution of the pores after sintering may be obtained.

In the case where the sintering in the sintering step is performed by an electro-discharge-sintering method, after a molded body is produced, a sintered body may be produced in a vacuum atmosphere. In this case, a degree of vacuum may be 1 to 5 Torr, preferably 3 to 5 Torr, and more preferably 4 or 5 Torr.

When the degree of vacuum is out of the corresponding range, a lot of time and energy are required to maintain the vacuum, which may cause an increase in production time and a decrease in productivity.

Thereafter, sintering is induced by applying an input energy to the molded body. The input energy at this time is preferably set to 0.1 to 0.5 kJ, preferably 0.1 to 0.4 kJ, and more preferably 0.1 to 0.3 kJ.

When the value of the input energy is out of the corresponding range, densification of the microstructure is induced, and thus, there are few pores 10 or a molded body formed of closed pores is formed, which significantly reduces fluidity of gas and fluid. Therefore, it is difficult to apply as a filter material. When a high value of the input energy is applied, an energy higher than a glass transition temperature of the amorphous powder particle is applied to cause formation of some crystals during solidification after the molding, which may cause degradation of physical and chemical properties of the component.

In addition, when the value of the input energy is lower than the corresponding value, sintering is not performed, and thus, the bonding portion between the alloy powder particles is not preferably formed. Therefore, the powder particle connection body 4 is not formed, or the amorphous metal porous body 100 is easily broken when being coupled to another component.

In the case of the electro-discharge-sintering method, the powder particles may be bonded and sintered by discharging the powder particles by applying a high voltage and current within a short time of microseconds, and it is possible to overcome the problems in which inherent microstructure and mechanical properties of the material and the ratio of the amorphous phases occurring in a high-temperature sintering method according to the related art are changed.

When the electro-discharge-sintering is performed, a high electrical energy may be applied to the powder particles for a very short time, portions of the surfaces of the amorphous alloy powder particles 1 may be rapidly melted and sintered to form a bonding portion in surface regions of the adjacent powder particles, and the process is performed at a temperature near the glass transition temperature ($T_g$), and thus, amorphous phases may be obtained at a high ratio.

Another aspect of the present disclosure is another method for producing an amorphous metal porous body 100, which is a method for producing a metal porous body by pressure sintering amorphous alloy powder particles. The method includes an amorphous alloy powder particle preparation step S21 and a pressure sintering step S22. Hereinafter, contents of the method for producing a metal porous body and the metal porous body that are the same as the contents described above may be included in the specification, and some repeated contents have been omitted.

The amorphous alloy powder particle preparation step S21 is a step of producing or preparing amorphous alloy powder particles having a desired composition. According to an exemplary embodiment in the present disclosure, the amorphous alloy powder particle may be an iron-based alloy containing at least one element selected from the group consisting of Cr, Mo, Co, Cu, Al, Ti, V, Si, Ni, P, Zn, Zr, Nb, Ag, Ta, Mg, Sn, W, Y, B, and C, and a balance of Fe, and in particular, an alloy having a composition having a high amorphous forming ability may be used.

The pressure sintering step S22 is a step of charging the prepared powder particles into a mold having a desired shape and pressure sintering the powder particles in a high temperature condition to bond particles of the amorphous metal powder particles and thus to form a foam.

Before performing the pressure sintering, it is possible to control a density of an amorphous porous sintered body by pressing the amorphous alloy powder particles, and the applied pressure may be differently controlled depending on the particle diameter and hardness of the amorphous powder particles, and may be 10 to 100 MPa and preferably 30 to 80 MPa.

A temperature during the pressure sintering is not limited, may be derived based on the properties of the amorphous powder particle, such as the composition, the microstructural structure, and the hardness, and may be selected according to a compression test of the metal porous body, observation of the shape of the bonding portion, and the distribution of the pores 10, after sintering. As an example, the temperature may be 500 to 1,200°C, 500 to 1,000°C, and preferably 500 to 800°C.

When the sintering temperature is too high, the bonding portions (necks) between the powder particles are excessively formed, and thus, the porosity of the metal porous body and the size of the pore may be reduced, segregation of some elements of additive elements may occur, and coarse crystalline phases may be formed. In addition, when the sintering temperature is too low, sufficient sintering is not performed, which causes degradation of the physical properties of the metal porous body.

The applied pressure during the pressure sintering may be controlled in various ranges according to the sintering temperature, and may be selected according to the observation of the shape of the bonding portion of the metal porous body, and the distribution and the sizes of the pores 10, after sintering. As an example, the applied pressure may be 10 to 100 MPa, 10 to 80 MPa, and preferably 10 to 50 MPa.

When the range of the applied pressure is too wide, a large number of bonding portions (necks) between the powder particles are formed, and thus, the porosity and the size are reduced, which may cause formation of a closed foam or formation of coarse crystalline phases. In addition, when the applied pressure is too low, thicknesses of the bonding portions (necks) between the powder particles are thin, and thus, the mechanical properties may be degraded, and it is difficult to maintain the shape of the component, resulting in breakage of the product.

As for a direction of the applied pressure, one-way compression, two-way compression, injection molding methods, and the like may be used, but are not limited thereto, and may be selectively used according to the microstructure (the porosity, the pore size, and the shape and the thickness of the bonding portion (neck)) for each position of the component after sintering.

As for the amorphous metal porous body, in addition to the electro-discharge-sintering and pressure sintering methods, it is also possible to use a method for producing a metal porous body by mixing the amorphous alloy powder particles 1 with a binder 11 and space holders and then performing sintering.

In a case where amorphous powder particles and space holders 2 are mixed and used, in the amorphous alloy powder particle preparation step S21, a step of mixing amorphous alloy powder particles 1 and space holders may be further included.

In addition, before the pressure sintering step S22, a step of preparing an intermediate molded body 3 using the amorphous alloy powder particles 1 and the space holders 2 may be included, and a space holder removal step may be included simultaneously with the pressure sintering step or before and after the pressure sintering step.

The space holders, which are particles mixed with the amorphous alloy powder particles and having a certain volume, form an intermediate molded body (green body) together with the amorphous alloy powder particles, and are removed in a subsequent process, and are materials for maintaining the plurality of pores 10 provided in the amorphous metal porous body 100. The pores 10 may be formed in a space occupied by the space holders.

In this case, materials of various compositions may be used for the space holders 2. The material is required to have chemical stability because it should not chemically react with the amorphous alloy powder particles, and it is preferable that the material has a property of easy removal so that the step of forming pores may be easily performed after being removed. It is preferable that the material has ductility at room temperature so as to preserve the positions of the pores by occupying a certain volume without being broken during production of the intermediate molded body 3.

A compound that may be used is not limited as long as the above conditions are satisfied, polymers such as polyethylene (PE), polypropylene (PP), polystyrene (PS), ethylene vinyl acetate, polyoxymethylene, carbohydrate, and sugars used in a metal injection molding (MIM) process may be used, a material such as ammonium carbonate or carbamide may be used, and a metal such as Mg or various salts including an inorganic salt of a metal such as NaCl may be used.

These space holders 2 may be removed using a subsequent heat treatment or liquid-phase leaching method, the pores 10 may be formed in the positions where the space holders 2 have been present, and the intermediate sintered body from which the space holders are removed may be produced into an amorphous foam through a subsequent heat treatment process or pressure sintering process.

The method for producing a metal porous body according to the present aspect of the present disclosure and the metal porous body produced by the method may implement excellent physical properties, elasticity, corrosion resistance, and lightweight. Thus, the metal porous body may be used in various material fields and fields of automobiles, flights, ships, interior materials, gas and fluid filter materials, and may also be used as a carrier, a support, a support material, and the like because it has an excellent surface area.

In addition, the metal porous body may be used as a membrane filter for separation, concentration, fractionation, isolation, fixation, and adsorption, a hydrogen storage alloy, a catalyst support, and the like.

Hereinafter, the contents of the present disclosure will be described in detail with reference to Examples.

**(Examples)**

Example 1 - Production of amorphous metal porous body

After amorphous alloy powder particles having a particle size distribution of 15 to 64 $\mu$m were prepared from an amorphous alloy to which Fe as a basic composition, 30.9 wt% of Cr, 4.0 wt% of Mo, 18.0 wt% of Ni, 10.0 wt% of Co, 2.5 wt% of Cu, 4.0 wt% of B, 1.4 wt% of Si, and a balance of Fe were added, the amorphous alloy powder particles were sintered by applying a pressure of 10 MPa under a condition of 1,000°C using a pressure sintering method, and the remaining conditions were set in the same manner as in Example 1, thereby producing a metal porous body.

Example 2

A metal porous body was produced in the same manner as in Example 1, except that amorphous alloy powder particles having a particle size distribution of 50 to 100 $\mu$m were used in Example 1.

Example 3

Sintering was performed under conditions of a degree of vacuum of 5 Torr and an input energy of 0.3 kJ using an electro-discharge-sintering method using amorphous alloy powder particles having a particle size distribution of 50 to 100 $\mu$m in Example 1.

Example 4

After amorphous alloy powder particles having a particle size distribution of 80 to 100 $\mu$m were prepared from an amorphous alloy to which Fe as a basic composition, 27.1 wt% of Mo, 15.8 wt% of Cr, 1.2 wt% of B, 3.7 wt% of C, and a balance of Fe were added, the amorphous alloy powder particles were sintered by applying a pressure of 10 MPa at a temperature of 500°C using a pressure sintering method, thereby producing a metal porous body.

Example 5

Sintering was performed under conditions of a degree of vacuum of 5 Torr and an input energy of 0.3 kJ using an electro-discharge-sintering method using the amorphous alloy powder particles in Example 4.

Example 6

Based on Example 1, after a mixed powder particle was prepared by mixing first amorphous alloy powder particles having a particle size of 50 to 100 $\mu$m and second amorphous alloy powder particles having a particle size of 15 to 50 $\mu$m at a weight ratio of 7:3, a pressure sintering method was used, and a pressure of 10 MPa was applied at a temperature of 1,000°C, thereby preparing a metal porous body.

Example 7

Based on Example 4, after a mixed powder particle was prepared by mixing first amorphous alloy powder particles having a particle size of 50 to 100 $\mu$m and second amorphous alloy powder particles having a particle size of 15 to 50 $\mu$m at a weight ratio of 7:3, the mixed powder particle was sintered under conditions of a degree of vacuum of 5 Torr and an input energy of 0.3 kJ using an electro-discharge-sintering method, thereby producing a metal porous body.

**(Comparative Examples)**

Comparative Example 1

A metal porous body was produced in the same manner as in Example 2, except that crystalline Fe-based powder particles (316L) were used instead of the amorphous alloy powder particles.

Comparative Example 2

A metal porous body was produced in the same manner as in Example 2, except that crystalline Ni-based powder particles (Hastelloy) were used instead of the amorphous alloy powder particles.

**(Experimental Examples)**

Experimental Example 1 - Physical property experiment of amorphous metal porous body

A pore diameter of a pore 10 included in the amorphous metal porous body produced in Example 2, bonding portions, and a microstructure were observed with FE-SEM (Tescan, MIRA 3), and the size and porosity of the pore 10 in the measured image were analyzed using an image analyzer. A density was measured using an archimedes method.

After a load was set to 10 g and a compression and decompression time was set to 10 sec, a measurement was performed using a vickers hardness test. The above results are shown in Table 1.

[Table 1]

| | Classification | First amorphous alloy powder particle particle size distribution ($\mu$m) | Second amorphous alloy powder particle particle size distribution ($\mu$m) | Average pore diameter ($\mu$m) | Cross section porosity (%) | Density (g/cm$^3$) | Hardness (Hv) |
|---|---|---|---|---|---|---|---|
| Example 1 | Semi-amorphous | 15 to 64 | - | 21.3 | 42.1 | 4.1 | 1023 |
| Example 2 | Semi-amorphous | 50 to 100 | - | 42.5 | 51.2 | 3.4 | 1120 |
| Example 3 | Semi-amorphous | 50 to 100 | - | 53.6 | 55.6 | 3.1 | 1137 |
| Example 4 | Amorphous | 80 to 100 | - | 41.2 | 50.3 | 3.6 | 1240 |
| Example 5 | Amorphous | 80 to 100 | - | 59.3 | 58.9 | 3.0 | 1260 |
| Example 6 | Semi-amorphous | 50 to 100 | 15 to 50 | 13.2 | 39.6 | 4.2 | 1115 |
| Example 7 | Amorphous | 50 to 100 | 15 to 50 | 14.1 | 38.5 | 4.4 | 1217 |
| Comparative Example 1 | Crystalline (316L) | 50 to 100 | - | 42.5 | 52.3 | 3.8 | 138 |
| Comparative Example 2 | Crystalline (Hastelloy) | 50 to 100 | - | 44.6 | 53.6 | 4.1 | 323 |

Experimental Example 2 - Corrosion resistance experiment of amorphous metal porous body

Samples were molded using the amorphous alloy powder particles of Examples 2 and 4 and Comparative Examples 1 and 2, and then a corrosion resistance test was performed. In the corrosion resistance test, corrosion properties were evaluated by measuring weights before and after corrosion by immersing the produced porous materials in an acidic solution at room temperature for 72 hours. At this time, an immersion test was performed in each of corrosive solutions such as 50% HF, 18.5% HCl, 49% $H_2SO_4$ solutions. The results are shown in Table 2.

[Table 2]

| | Corrosive reagent | Weight of sample before corrosion (g) | Weight of sample after corrosion (g) | Corrosion loss rate (%) |
|---|---|---|---|---|
| Example 2 | 50% HF | 5.2591 | 4.7463 | 9.75 |
| | 49% $H_2SO_4$ | 2.4362 | 2.4365 | -0.01 |
| | 18.5% HCl | 3.3138 | 3.0350 | 8.41 |
| Example 4 | 50% HF | 6.7331 | 6.5893 | 1.94 |
| | 49% $H_2SO_4$ | 10.7915 | 10.7952 | 0.03 |
| | 18.5% HCl | 10.9226 | 10.9229 | 0.00 |
| Comparative Example 1 | 50% HF | - | - | - |
| | 49% $H_2SO_4$ | 7.7767 | 7.7723 | 0.06 |
| | 18.5% HCl | 6.2237 | 5.4625 | 12.23 |

(continued)

| | Corrosive reagent | Weight of sample before corrosion (g) | Weight of sample after corrosion (g) | Corrosion loss rate (%) |
|---|---|---|---|---|
| Comparative Example 2 | 50% HF | 6.3634 | 5.5244 | 13.18 |
| | 49% $H_2SO_4$ | 7.2656 | 7.2653 | 0.00 |
| | 18.5% HCl | 7.2252 | 7.2189 | 0.09 |

In Example 2, the corrosion loss rate was significantly low under the acid conditions of hydrofluoric acid, sulfuric acid, and hydrochloric acid, and the corrosion rate was significantly low in sulfuric acid and hydrochloric acid. Therefore, it can be confirmed that the amorphous metal porous body may be used even in a corrosive environment under acidic conditions. In Examples 2 and 4, the corrosion loss rate was significantly low under the acidic conditions of hydrofluoric acid, sulfuric acid, and hydrochloric acid. In particular, in Example 4, the corrosion loss rate was significantly low in hydrofluoric acid, sulfuric acid, and hydrochloric acid in comparison to the existing crystalline material, and it was confirmed that the amorphous metal porous body could be used even in a corrosive environment under acidic conditions. In particular, a highly corrosion resistant amorphous metal porous body having a corrosion loss rate of less than 2.0% at 50% HF was obtained.

The features, structure, effects, and the like exemplified in each exemplary embodiment described above may be combined with another exemplary embodiment or may be changed by those skilled in the art to which the exemplary embodiments pertain. Therefore, it is to be understood that contents associated with such combination or change fall within the scope of the present disclosure.

(Description of Reference Numerals)

1: Amorphous alloy powder particle
2: Space holder
4: Powder particle connection body
10: Pore
100: Amorphous metal porous body

Claims

1. An amorphous metal porous body that is a metal porous body including pores, comprising:

powder particle connection bodies in which at least portions of amorphous alloy powder particles adjacent to each other are connected in a network structure; and
a plurality of pores provided between the powder particle connection bodies.

2. The amorphous metal porous body of claim 1, wherein the powder particle connection body includes a bonding portion (neck) at which portions of surfaces of the adjacent amorphous alloy powder particles are fused and bonded to each other.

3. The amorphous metal porous body of claim 1, wherein the amorphous metal porous body has a corrosion loss rate of 10.0% or less under a condition of 50% HF.

4. The amorphous metal porous body of claim 2, wherein the amorphous alloy powder particle is connected to adjacent alloy powder particles at at least two bonding portions to form the network structure.

5. The amorphous metal porous body of claim 1, wherein a hardness of the amorphous metal porous body is 700 to 1,300 Hv.

6. The amorphous metal porous body of claim 1, wherein a density of the amorphous metal porous body is 1.5 to 7.0 g/cm$^2$.

7. The amorphous metal porous body of claim 1, wherein a uniformity of the pores is 1 to 30.

8. The amorphous metal porous body of claim 1, wherein the amorphous alloy powder particle includes an iron-based alloy having at least one element selected from the group consisting of Cr, Mo, Co, Cu, Al, Ti, V, Si, Ni, P, Zn, Zr, Nb, Ag, Ta, Mg, Sn, W, Y, B, and C, and Fe.

9. The amorphous metal porous body of claim 1, wherein the amorphous alloy powder particles include first and second amorphous alloy powder particles having different average particle diameters.

10. A method for producing an amorphous metal porous body, the method comprising:

   a powder particle preparation step of preparing amorphous alloy powder particles containing amorphous phases; and
   a sintering step of supplying an input energy to the amorphous alloy powder particles to form powder particle connection bodies in which amorphous alloy powder particles adjacent to each other are connected in a network structure.

11. The method of claim 10, wherein the input energy is an electric energy.

12. The method of claim 11, wherein the powder particle connection body includes the amorphous alloy powder particles that are adjacent to each other and have portions of surfaces connected to each other at an outer face, and a distance (d) between the centers of the amorphous alloy powder particles satisfies the following Expression 1:

$$\text{(Expression 1)}$$

$$0.5 \times (D_A + D_B)/2 \leq d \leq 1.1 \times (D_A + D_B)/2$$

   wherein each of $D_A$ and $D_B$ is a particle diameter of each of the adjacent amorphous alloy powder particles.

13. The method of claim 11, wherein the amorphous alloy powder particles include first and second amorphous alloy powder particles having different average particle diameters.

14. The method of claim 11, wherein the input energy is 0.1 to 0.5 kJ.

15. A method for producing an amorphous metal porous body, the method comprising:

   a powder particle preparation step of preparing amorphous alloy powder particles containing amorphous phases; and
   a sintering step of applying a temperature and a pressure to the amorphous alloy powder particles to form powder particle connection bodies in which amorphous alloy powder particles adjacent to each other are connected in a network structure.

16. The method of claim 15, wherein the powder particle connection body includes the amorphous alloy powder particles that are adjacent to each other and have portions of surfaces connected to each other at an outer face, and a distance (d) between the centers of the amorphous alloy powder particles satisfies the following Expression 1:

$$\text{(Expression 1)}$$

$$0.5 \times (D_A + D_B)/2 \leq d \leq 1.1 \times (D_A + D_B)/2$$

   wherein each of $D_A$ and $D_B$ is a particle diameter of each of the adjacent amorphous alloy powder particles.

17. The method of claim 15, wherein the amorphous alloy powder particles include first and second amorphous alloy powder particles having different average particle diameters.

18. The method of claim 15, wherein in the sintering, the temperature is 400°C or higher and 1,200°C or lower, and the pressure is 10 MPa or more and 100 MPa or less.

19. The method of claim 15, wherein the powder particle preparation step further includes a step of mixing space holders having a composition different from that of the amorphous alloy powder particle with the amorphous alloy powder particles.

20. The method of claim 19, wherein the space holder includes one or two or more materials selected from the group consisting of polyethylene, polypropylene, polystyrene (PS), ethylene vinyl acetate, polyoxymethylene, carbohydrate, ammonium carbonate, carbamide, a metal, and an inorganic metal salt.

【Fig. 1】

100

【Fig. 2】

(a)                    (b)                    (c)

【Fig. 3】

【Fig. 4】

<div align="center">**INTERNATIONAL SEARCH REPORT**</div>

| International application No. |
|---|
| **PCT/KR2021/000354** |

**A. CLASSIFICATION OF SUBJECT MATTER**

**A61L 27/04**(2006.01)i; **A61C 8/00**(2006.01)i; **A61K 6/84**(2020.01)i

According to International Patent Classification (IPC) or to both national classification and IPC

**B. FIELDS SEARCHED**

Minimum documentation searched (classification system followed by classification symbols)

A61L 27/04(2006.01); B01J 19/08(2006.01); B22F 1/00(2006.01); B22F 3/10(2006.01); B22F 3/11(2006.01); B22F 3/14(2006.01); B22F 9/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 금속다공체(metallic foam), 비정질 합금(amorphous alloy), 분말(powder), 기공(pore), 네트워크(network), 분말연결체(powder connecting body), 접합부(neck), 입력에너지(input energy), 온도(temperature), 압력(pressure), 소결(sintering)

**C. DOCUMENTS CONSIDERED TO BE RELEVANT**

| Category* | Citation of document, with indication, where appropriate, of the relevant passages | Relevant to claim No. |
|---|---|---|
| X | KR 10-2013-0109545 A (INDUSTRY ACADEMY COOPERATION FOUNDATION OF SEJONG UNIVERSITY) 08 October 2013 (2013-10-08) See paragraphs [0015]-[0022] and [0030]-[0041]; and figure 3. | 1-7,9-14 |
| Y | | 8,15-20 |
| Y | JP 09-078108 A (NISSHIN STEEL CO., LTD.) 25 March 1997 (1997-03-25) See abstract; and paragraphs [0011]-[0013], example 1. | 8 |
| Y | JP 2011-001587 A (NISSAN MOTOR CO., LTD.) 06 January 2011 (2011-01-06) See abstract; and paragraphs [0016]-[0027]. | 15-20 |
| Y | KR 10-2014-0140327 A (KOREA INSTITUTE OF INDUSTRIAL TECHNOLOGY) 09 December 2014 (2014-12-09) See abstract; and claims 1-14. | 19-20 |

☑ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **27 April 2021** | **27 April 2021** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2021/000354** |

C.    DOCUMENTS CONSIDERED TO BE RELEVANT

| Category* | Citation of document, with indication, where appropriate, of the relevant passages | Relevant to claim No. |
| --- | --- | --- |
| A | 손창영 등. Fe계 비정질 합금 분말을 고에너지 전자빔으로 투사하여 제조된 표면복합재료의 경도와 내식성. 대한금속재료학회지. 2006, vol. 44, no. 1, pp. 30-36 (SON, Chang Young et al. Hardness and Corrosion Resistance of Surface Composites Fabricated by High-energy Electron-beam Irradiation of Fe-based Amorphous Alloy Powders. The journal of The Korean Institute of Metals and Materials.).<br>See entire document. | 1-20 |

**INTERNATIONAL SEARCH REPORT**
Information on patent family members

International application No.

**PCT/KR2021/000354**

| Patent document cited in search report | | | Publication date (day/month/year) | Patent family member(s) | | | Publication date (day/month/year) |
|---|---|---|---|---|---|---|---|
| KR | 10-2013-0109545 | A | 08 October 2013 | KR | 10-1566054 | B1 | 06 November 2015 |
| JP | 09-078108 | A | 25 March 1997 | None | | | |
| JP | 2011-001587 | A | 06 January 2011 | None | | | |
| KR | 10-2014-0140327 | A | 09 December 2014 | KR | 10-1501765 | B1 | 11 March 2015 |

Form PCT/ISA/210 (patent family annex) (July 2019)